# EUROPEAN PATENT APPLICATION

(11) **EP 2 319 514 A1**
(43) Date of publication of application: **11.05.2011**
(21) Application number: 09800231.4
(22) Date of filing: 24.07.2009
(51) Int. Cl.: A61K 31/4725, A61K 9/08, A61K 47/04, A61K 47/12, A61K 47/18, A61P 27/02

(54) **STABLE AQUEOUS SOLUTION COMPOSITION CONTAINING SULFONAMIDE COMPOUND**

(30) Priority: 25.07.2008 JP 2008192299
(71) Applicant: Asahi Kasei Pharma Corporation, Tokyo 101-8101 (JP)
(72) Inventor: KANZAWA, Yoshihito, Tokyo 101-8101 (JP); KATAYAMA, Kazuhiko, Tokyo 101-8101 (JP); NISHIO, Fumihide, Tokyo 101-8101 (JP)
(74) Representative: Forstmeyer, Dietmar
(86) International application number: PCT/JP2009/003487
(87) International publication number: WO 2010/010715

(57) **Abstract**

An aqueous solution composition containing (S)-1-(4-chloro-5-isoquinolinesulfonyl)-3-aminopyrrolidine or (S)-1-(4-chloro-5-isoquinohnesulfonyl)-3-(methylamino)pyrrolidine as an active ingredient, and not containing citric acid or a salt thereof, which has high stability so as to be storable at room temperature.

## Description

### Technical Field

The present invention relates to an aqueous solution composition containing a sulfonamide compound or a pharmacologically acceptable salt thereof having superior intraocular pressure reducing action, which has high stability so as to be storable at room temperature.

### Background Art

In the eyeball under healthy condition, aqueous humor continuously circulates to maintain a certain level of intraocular pressure. However, when the flow rate of aqueous humor at the trabeculum as the outlet of aqueous humor gradually decreases, or the chamber angle becomes narrow to disturb the flow of aqueous humor, the intraocular pressure in the eyeball increases, which results in the onset of glaucoma in which oppression of the optic nerve causes abnormal visual field, ocular hypertension which is not accompanied by abnormal visual field but highly possibly develops into glaucoma after a prolonged period of time, and the like. For the therapeutic treatment of these glaucoma and ocular hypertension, it is necessary to reduce the increased intraocular pressure. Practically in the clinical field, sympathomimetics such as epinephrine, parasympathomimetics such as pilocarpine hydrochloride, beta blockers such as timolol, prostaglandin agents such as isopropyl unoprostone, carbonate dehydrarase inhibitors such as dorzolamide, and the like have been used. However, any of these therapeutic drugs are not satisfactory from a viewpoint of intraocular pressure reducing action.

For a purpose of enhancing intraocular pressure reducing action, a therapeutic treatment based on a combination of a beta blocker, a prostaglandin agent, and a carbonate dehydrarase inhibitor (see, Patent document 1), and a method for maintaining intraocular pressure reducing action on the basis of a combination of a beta blocker and alginic acid (see, Non-patent document 1) have recently been also reported. More recently, it has been reported that compounds having Rho kinase inhibitory activity have superior therapeutic and prophylactic effects for glaucoma, and have potent intraocular pressure reducing action (see, Patent document 2). There have also been reported methods of enhancing intraocular pressure reducing action by using a combination of a compound having Rho kinase inhibitory activity and phosphoric acid or boric acid (see, Patent documents 3 and 4).

Among patients with glaucoma, lots of patients develop glaucoma without the increase of intraocular pressure (normal intraocular pressure glaucoma), and for the treatment of such patients, normal intraocular pressure needs to be further reduced. However, it is still more difficult to reduce normal intraocular pressure than to reduce increased intraocular pressure, and therapeutic treatments of normal intraocular pressure glaucoma by using the aforementioned available medicaments and combinations thereof are limited. Therefore, it is desired by those in practical clinical filed to provide a medicament having more excellent intraocular pressure reducing action. Moreover, polypharmacy has a problem of lower compliance of patients, and it is desired by those in practical clinical field to provide a therapeutic agent that can achieve intraocular pressure reducing effect in combination with medicaments as fewer as possible.

It is also known that (S)-1-(4-chloro-5-isoquinolinesulfonyl)-3-aminopyrrolidine or a salt thereof, or (S)-1-(4-chloro-5-isoquinolinesulfonyl)-3-(methylamino)pyrrolidine or a salt thereof has intraocular pressure reducing action and neutrophil migration inhibitory action based on inhibition of phosphorylation of the myosin regulatory light chain, and is useful as a prophylactic and/or therapeutic agent for glaucoma and the like (see, Patent document 5).

### Prior art references

### Patent documents

Patent document 1: Japanese Patent Unexamined Publication based on PCT Application (KOHYO) No. 2002-511430
Patent document 2: International Patent Publication WO00/9162
Patent document 3: International Patent Publication WO06/68208
Patent document 4: Japanese Patent Unexamined Publication (KOKAI) No. 2006-290827
Patent document 5: International Patent Publication WO2007/026664

### Non-patent document

Non-patent document 1: Practical Ophthalmology, 4(5):2-6, 2001

### Summary of the Invention

### Object to be Achieved by the Invention

The inventors of the present invention conducted various researches on eye drops in the form of aqueous solution containing (S)-1-(4-chloro-5-isoquinolinesulfonyl)-3-aminopyrrolidine or a salt thereof, or (S)-1-(4-chloro-5-isoquinolinesulfonyl)-3-(methylamino)pyrrolidine or a salt thereof mentioned above as an active ingredient, and in the course of these researches, they faced a problem that the aforementioned active ingredient was unstable in a certain type of an aqueous solution composition, and when the aqueous solution composition was stored at room temperature, decomposition of the active ingredient advanced. In order to provide a medicament containing the aforementioned active ingredient for use in the practical clinical field, it is necessary to prepare an aqueous solution composition stable for a prolonged period of time even when stored at room temperature.

Therefore, an object of the present invention is to provide an aqueous solution composition containing one kind or two or more kinds of substances selected from the group consisting of (S)-1-(4-chloro-5-isoquinohnesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, and having high stability so as to be storable at room temperature.

### Means for Achieving the Object

The inventors of the present invention conducted various researches in order to achieve the aforementioned object. As a result, they detected citric acid or a salt thereof as a substance that degraded stability of the aforementioned substances as the active ingredient, and found that when citric acid or a salt thereof was allowed to coexist in an aqueous solution composition, long-term storage stability of the aqueous solution composition was markedly degraded, and that a stable aqueous solution composition was successfully provided by preparing an aqueous solution composition without using citric acid or a salt thereof. The inventors of the present invention also detected a pH range in which the aqueous solution was capable of being stably maintained, and found that intraocular pressure reducing action of the aforementioned active ingredient was enhanced in said pH range.

Furthermore, the inventors of the present invention also faced a problem that the aforementioned active ingredient was decomposed by light irradiation at 5,000 lux for ten days (1,200,000 lux -hr) in a solution containing 0.9% of sodium chloride (hereinafter in the specification, said solution may also be referred to as "physiological saline solution"). The inventors found that the aqueous solution composition absorbed lights having a wavelength of 350 nm or shorter, whilst hardly absorbed lights having a wavelength larger than 350 nm, and that the aqueous solution composition was successfully stored stably over a long period of time by preventing irradiation with ultraviolet lights having a wavelength of 350 nm or shorter, and the aqueous solution composition was stably distributed and stored over a long period of time by applying a package having such particular characteristics.
The present invention was accomplished on the basis of these findings.

The present invention thus relates to the followings:
(1) An aqueous solution composition containing one or two or more of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrohdine and a pharmacologically acceptable salt thereof as an active ingredient, and not containing citric acid or a salt thereof;
(2) The aqueous solution composition according to (1) mentioned above, which contains (S)-1-(4-chloro-5-isoquinolinesulfonyl)-3-aminopyrrolidine or a pharmacologically acceptable salt thereof as an active ingredient;
(3) The aqueous solution composition according to (1) mentioned above, which contains (S)-1-(4-chloro-5-isoquinolinesulfonyl)-3-(methylamino)pyrrolidine or a pharmacologically acceptable salt thereof as an active ingredient;

(4) The aqueous solution composition according to any one of (1) to (3) mentioned above, wherein the pharmacologically acceptable salt is a hydrogen halide acid salt;
(5) The aqueous solution composition according to (4) mentioned above, wherein the pharmacologically acceptable salt is monohydrochloride;
(6) The aqueous solution composition according to any one of (1) to (5) mentioned above, wherein the aqueous solution has a pH of 5 to 9;
(7) The aqueous solution composition according to (6) mentioned above, which further contains a pH adjustor other than citric acid or a salt thereof;
(8) The aqueous solution composition according to (7) mentioned above, wherein the pH adjustor other than citric acid or a salt thereof consists of one kind or two or more kinds of pH adjustors selected from the group consisting of phosphoric acid and a salt thereof, acetic acid and a salt thereof, tris(hydroxymethyl)aminomethane and a salt thereof, as well as boric acid and a salt thereof;

(9) The aqueous solution composition according to (7) or (8) mentioned above, wherein a ratio of molar concentration of the pH adjustor and a total of molar concentrations of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof is 1:10 to 200:1;
(10) An aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, wherein the aqueous solution has a pH of 7 to 9;

(11) The aqueous solution composition according to (10) mentioned above, wherein the aqueous solution has a pH of 7 to 9;
(12) A method for stabilizing an aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, which comprises the step of adjusting a pH of the aqueous solution composition to 5 to 9 in the absence of citric acid or a salt thereof by using a pH adjustor other than citric acid or a salt thereof;

(13) A method for preparing a long-term storable aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, which comprises the step of adjusting a pH of the aqueous solution composition to 5 to 9 by using a pH adjustor in the absence of citric acid or a salt thereof;
(14) A method for preparing a pharmaceutical composition wherein intraocular pressure reducing action of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof is enhanced , which comprises preparing an aqueous solution composition having a pH of 7 to 9 using the substance;

(15) A method for enhancing intraocular pressure reducing action of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinohnesulfonyl)-3-(methylamino)pyrrohdine and a pharmacologically acceptable salt thereof, which comprises the use of the substance as an aqueous solution having a pH of 7 to 9;
(16) A method for preparing a long-term storable aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof having enhanced intraocular pressure reducing action, which comprises preparing the substance as an aqueous solution composition having a pH of 7 to 9 by using a pH adjustor in the absence of citric acid or a salt thereof;

(17) An aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, which is filled in a container having a permeability of 40% or lower for ultraviolet lights having a wavelength of 350 nm or shorter, and/or filled in a container stored in a packaging container having a permeability of 20% or lower for ultraviolet lights having a wavelength of 350 nm or shorter;
(18) An aqueous solution composition for instillation containing (S)-1-(4-chloro-5-isoquinolinesulfonyl)-3-aminopyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, and having a pH of 7 to 9 wherein citric acid or a salt thereof is not contained, which is filled in a container having a permeability of 40% or lower for ultraviolet lights having a wavelength of 350 nm or shorter, and/or filled in a container stored in a packaging container having a permeability of 20% or lower for ultraviolet lights having a wavelength of 350 nm or shorter;

(19) An aqueous solution composition for instillation containing (S)-1-(4-chloro-5-isoquinolinesulfonyl)-3-(methylamino)pyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, not containing citric acid or a salt thereof, and having a pH of 7 to 9, which is filled in a container having a permeability of 40% or lower for ultraviolet lights having a wavelength of 350 nm or shorter, and/or filled in a container stored in a packaging container having a permeability of 20% or lower for ultraviolet lights having a wavelength of 350 nm or shorter;
(20) An aqueous solution composition for instillation containing (S)-1-(4-chloro-5-isoquinolinesulfonyl)-3-aminopyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, and having a pH of 7 to 9 wherein citric acid or a salt thereof is not contained, which is filled in a plastic container having a permeability of 40% or lower for ultraviolet lights having a wavelength of 350 nm or shorter, and/or filled in a container stored in a packaging container having a permeability of 20% or lower for ultraviolet lights having a wavelength of 350 nm or shorter;

(21) An aqueous solution composition for instillation containing (S)-1-(4-chloro-5-isoquinolinesulfonyl)-3-(methylamino)pyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, and having a pH of 7 to 9 wherein citric acid or a salt thereof is not contained, which is filled in a plastic container having a permeability of 40% or lower for ultraviolet lights having a wavelength of 350 nm or shorter, and/or filled in a container stored in a packaging container having a permeability of 20% or lower for ultraviolet lights having a wavelength of 350 nm or shorter;
(22) The aqueous solution composition according to any one of (1) to (21) mentioned above, which is filled in a container having a permeability of 40% or lower for ultraviolet lights having a wavelength of 350 nm or shorter, and/or filled in a container stored in a packaging container having a permeability of 20% or lower, preferably 10% or lower, for ultraviolet lights having a wavelength of 350 nm or shorter;

(23) The aqueous solution composition according to (22) mentioned above, wherein the container is a container for instillation;
(24) The aqueous solution composition according to (22) mentioned above, wherein the container in which the aqueous solution composition is filled is a container for instillation;
(25) The aqueous solution composition according to (22), (23), or (24) mentioned above, wherein the container in which the aqueous solution composition is filled is made of plastics;

(26) A method for stabilizing an aqueous solution composition against light which contains one kind or two or more kinds of substances selected from the group consisting of (S)-1-(4-chloro-5-isoquinohnesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, which comprises the step of filling the aqueous solution composition in a container having a permeability of 40% or lower for ultraviolet lights having a wavelength of 350 nm or shorter, and/or the step of filling the aqueous solution composition in a container stored in a packaging container having a permeability of 20% or lower for ultraviolet lights having a wavelength of 350 nm or shorter;
(27) A method for stabilizing the aqueous solution composition according to any one of (1) to (11) mentioned above against light, which comprises the step of filling the aqueous solution composition in a container having a permeability of 40% or lower for ultraviolet lights having a wavelength of 350 nm or shorter, and/or the step of filling the aqueous solution composition in a container stored in a packaging container having a permeability of 20% or lower, preferably 10% or lower, for ultraviolet lights having a wavelength of 350 nm or shorter;

(28) A method for stabilizing an aqueous solution composition for instillation against light which contains (S)-1-(4-chloro-5-isoquinolinesulfonyl)-3-aminopyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, and has a pH of 7 to 9 wherein citric acid or a salt thereof is not contained, which comprises the step of filling the aqueous solution composition in a container having a permeability of 40% or lower for ultraviolet lights having a wavelength of 350 nm or shorter, and/or the step of filling the aqueous solution composition in a container stored in a packaging container having a permeability of 20% or lower for ultraviolet lights having a wavelength of 350 nm or shorter;
(29) A method for stabilizing an aqueous solution composition for instillation against light which contains (S)-1-(4-chloro-5-isoquinolinesulfonyl)-3-(methylamino)pyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, and has a pH of 7 to 9 wherein citric acid or a salt thereof is not contained, which comprises the step of filling the aqueous solution composition in a container having a permeability of 40% or lower for ultraviolet lights having a wavelength of 350 nm or shorter, and/or the step of filling the aqueous solution composition in a container stored in a packaging container having a permeability of 20% or lower for ultraviolet lights having a wavelength of 350 nm or shorter;

(30) A method for stabilizing an aqueous solution composition for instillation against light which contains (S)-1-(4-chloro-5-isoquinolinesulfonyl)-3-aminopyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, and has a pH of 7 to 9 wherein citric acid or a salt thereof is not contained, which comprises the step of filling the aqueous solution composition in a plastic container having a permeability of 40% or lower for ultraviolet lights having a wavelength of 350 nm or shorter, and/or the step of filling the aqueous solution composition in a container stored in a packaging container having a permeability of 20% or lower for ultraviolet lights having a wavelength of 350 nm or shorter;
(31) A method for stabilizing an aqueous solution composition for instillation against light which contains (S)-1-(4-chloro-5-isoquinolinesulfonyl)-3-(methylamino)pyrrolidine or a pharmacologically acceptable salt thereof, together with phosphoric acid or a salt thereof, boric acid or a salt thereof, tris(hydroxymethyl)aminomethane or a salt thereof, or acetic acid or a salt thereof, and has a pH of 7 to 9 wherein citric acid or a salt thereof is not contained, which comprises the step of filling the aqueous solution composition in a plastic container having a permeability of 40% or lower for ultraviolet lights having a wavelength of 350 nm or shorter, and/or the step of filling the aqueous solution composition in a container stored in a packaging container having a permeability of 20% or lower for ultraviolet lights having a wavelength of 350 nm or shorter.

### Effect of the Invention

The aforementioned aqueous solution composition provided by the present invention, preferably the aqueous solution composition according to any one of (8) to (10) mentioned above, has superior properties that the composition can be stably stored at room temperature over a long period of time, and the intraocular pressure reducing action of the active ingredient is enhanced. Therefore, when the aqueous solution composition of the present invention is clinically used as a preparation for instillation, the composition is advantageous, because it does not require time for dissolving a solid preparation upon use, and can be immediately administered to a patient, and the composition can achieve prophylactic and/or therapeutic effect against glaucoma or ocular hypertension with a low administration frequency, because the composition has the enhanced intraocular pressure reducing action. According to another embodiment, by diluting the aqueous solution composition of the present invention to an appropriate concentration with an appropriate diluent before use, it becomes possible to administer the composition by instillation. Further, increase of a pH value of the aqueous solution and decrease of content of the active ingredient due to decomposition of the active ingredient during storage are also suppressed.

Furthermore, by filling the aforementioned aqueous solution composition in a container having a permeability of 40% or lower for ultraviolet lights having a wavelength of 350 nm or shorter, and/or filling the aqueous solution composition in a container stored in a packaging container having a permeability of 20% or lower, preferably 10% or lower, for ultraviolet lights having a wavelength of 350 nm or shorter, decomposition of the active ingredient by exposure to light at the time of storage, distribution, and use can be remarkably suppressed, and it becomes possible to more stably provide the aqueous solution composition as a medicament.

### Brief Description of the Drawings

[Fig. 1] Fig. 1 shows results of an intraocular pressure reducing test by instillation of a (S)-1-(4-chloro-5-isoquinolinesulfonyl)-3-(methylamino)pyrrolidine aqueous solution to rabbits.

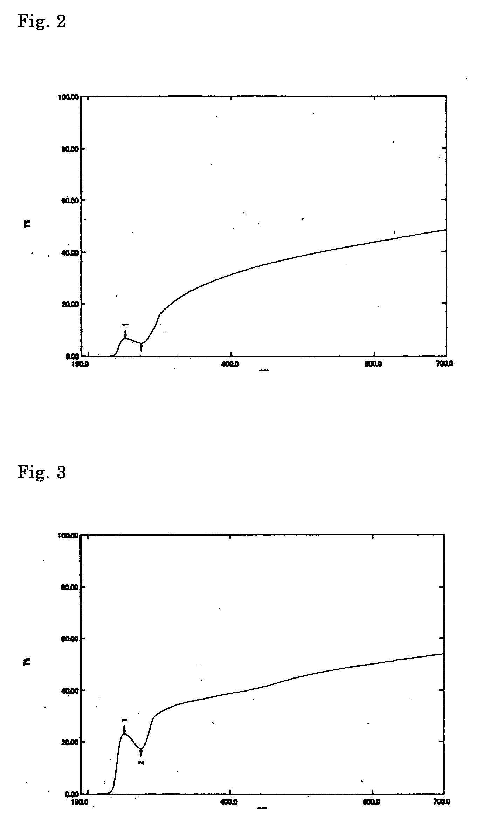

[Fig. 2] Fig. 2 shows a permeability spectrum of a white light-shielding container for 190 to 700 nm.
[Fig. 3] Fig. 3 shows a permeability spectrum of a yellow light-shielding container for 190 to 700 nm.

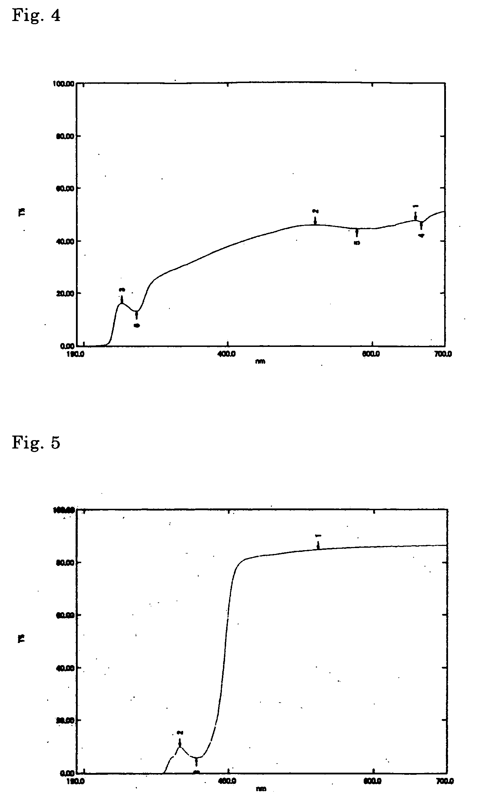

[Fig. 4] Fig. 4 shows a permeability spectrum of a blue light-shielding container for 190 to 700 nm.
[Fig. 5] Fig. 5 shows a permeability spectrum of a transparent ultraviolet-shielding bag for 190 to 700 nm.

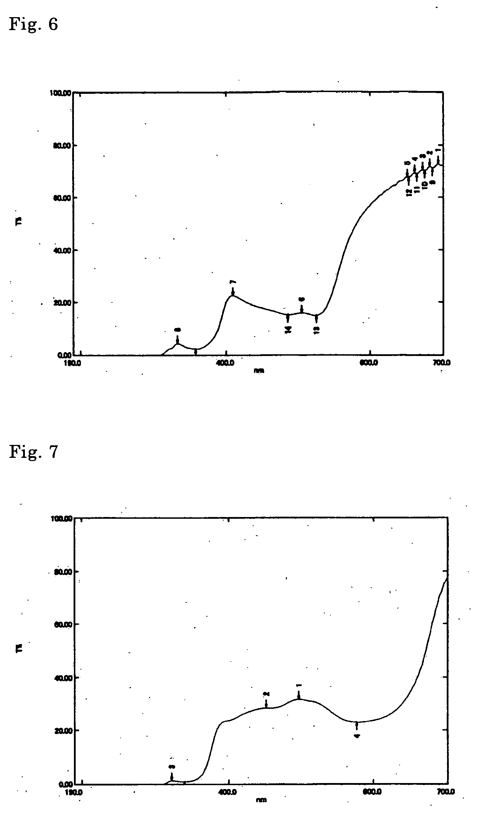

[Fig. 6] Fig. 6 shows a permeability spectrum of an orange ultraviolet-shielding bag for 190 to 700 nm.
[Fig. 7] Fig. 7 shows a permeability spectrum of a gray ultraviolet-shielding bag for 190 to 700 nm.

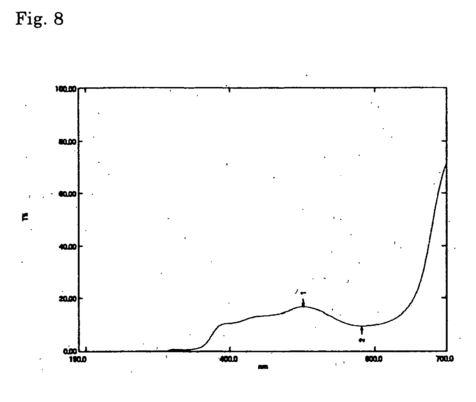

[Fig. 8] Fig. 8 shows a permeability spectrum of a dark gray ultraviolet-shielding bag for 190 to 700 nm.

### Modes for Carrying out the Invention

(S)-1-(4-Chloro-5-isoquinolinesulfonyl)-3-aminopyrrolidine and (S)-1-(4-chloro-5-isoquinolinesulfonyl)-3-(methylamino)pyrrolidine used as the active ingredient of the aqueous solution composition of the present invention can be prepared by the method described in International Patent Publication WO2007/026664. The aforementioned compounds are desirably used in the form of a pharmacologically acceptable salt. Examples of the salt include conventional non-toxic salts including mineral acid salts (e.g., hydrochloride, hydrobromide, sulfate, nitrate, phosphate), organic acid salts (e.g., acetate, methanesulfonate, tosylate), and the like. Among them, hydrogen halide acid salts (e.g., hydrochloride, hydrobromide) are preferred, and hydrochloride is especially preferred. The compounds in free form or pharmacologically acceptable salts thereof may form a hydrate or solvate. Number of water or solvent molecules to be added is not particularly limited, and arbitrary hydrates or solvates can be used.

In the case of a salt with a monovalent mineral acid or organic acid, up to two mineral acid or organic acid molecules can be added. The number of monovalent mineral acid or organic acid molecule to be added is preferably 1. In the case of a salt with a divalent mineral acid or organic acid, the number of divalent mineral acid molecule to be added is 1 or 1/2, and 1/2 is preferred. Most preferred salt is monohydrochloride. For example, (S)-1-(4-chloro-5-isoquinolinesulfonyl)-3-aminopyrrolidine monohydrochloride and/or (S)-1-(4-chloro-5-isoquinolinesulfonyl)-3-(methylamino)pyrrolidine monohydrochloride is preferred, and (S)-1-(4-chloro-5-isoquinolinesulfonyl)-3-(methylamino)pyrrolidine monohydrochloride is particularly preferred. There is also another embodiment in which (S)-1-(4-chloro-5-isoquinolinesulfonyl)-3-aminopyrrolidine monohydrochloride is particularly preferred. When a salt comprising two monovalent mineral acid or organic acid molecules added, or a salt comprising one divalent mineral acid or organic acid added is used for the present invention, it is preferable to relatively quickly prepare the aqueous solution composition of the present invention.

As a preferred embodiment of the aqueous solution composition of the present invention, an embodiment is preferred in which a ratio of total mass of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinohnesulfonyl-3-(methylamino)pyrrolidine and a pharmacologically acceptable salt thereof based on the total volume of the aqueous solution composition is 0.001 to 5 w/v%, and the ratio is more preferably 0.003 to 0.5 w/v%, particularly preferably 0.005 to 0.5 w/v%.
As another embodiment of the aqueous solution composition of the present invention, an embodiment is preferred in which a lower limit of the total of molar concentrations of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof is 0.03 mM or more, an embodiment is more preferred in which the lower limit is 0.08 mM or more, and an embodiment is particularly preferred in which the lower limit is 0.14 mM or more. As for an upper limit of said total, an embodiment includes in which the limit is not higher than a saturation solubility of said compound in the aqueous solution, and an embodiment is preferred in which the limit is 140 mM or lower, and an embodiment is particularly preferred in which the limit is 14 mM or lower.

The aqueous solution composition of the present invention is an aqueous solution composition not containing citric acid or a salt thereof, and a pH adjustor other than citric acid or a salt thereof can be used for preparation of the aqueous solution composition of the present invention. As the pH adjustor, acids, bases, and various buffering agents can be used. Examples include, for example, phosphoric acid or a salt thereof, boric acid or a salt thereof, tris(hydroxymethyl)aminomethane or a salt thereof, acetic acid or a salt thereof, and the like. Phosphoric acid or a salt thereof, tris(hydroxymethyl)aminomethane or a salt thereof, and boric acid or a salt thereof are preferred, and phosphoric acid or a salt thereof is particularly preferred. There is also another embodiment in which tris(hydroxymethyl)aminomethane or a salt thereof is particularly preferred. There is still another embodiment in which boric acid or a salt thereof is preferred.

Amount of the pH adjustor to be added can be appropriately chosen depending on a desired pH and a volume of the solution. pH of the aqueous solution composition of the present invention is preferably 5 to 9 from a viewpoint of stability of the aqueous solution composition. Further, from a viewpoint of enhancement of the intraocular pressure reducing action, pH is preferably 7 or higher, and most preferably 8 to 9. Therefore, as for pH of the aqueous solution composition of the present invention, a pH range of from 7 to 9 is particularly preferred, and a pH range of from 8 to 9 is most preferred. Ratio of a molar concentration of the pH adjustor and a total of molar concentrations of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof is preferably 1:10 to 400:1, more preferably 1:2 to 400:1, still more preferably 1:2 to 200:1, particularly preferably 2:1 to 30:1, and most particularly preferably 2:1 to 20:1. In a preferred embodiment, the ratio is around 18:1. When the ratio of the molar concentration of the pH adjustor and the total of molar concentrations of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof is around 1:10 to 5:1, pH may shift to an acidic side than a desired pH. In that case, the molar concentration ratio may be increased above the aforementioned range, or pH may be adjusted by using an alkali. The alkali is preferably used in the form of a solid or an aqueous solution at a high concentration.

The aqueous solution composition of the present invention may contain, besides the aforementioned pH adjustor, one kind or two or more kinds of pharmaceutical additives selected from isotonic agent, thickener, preservative, and the like, if necessary.
Examples of the isotonic agent include sodium chloride, potassium chloride, and the like. Examples of the thickener include methylcellulose, carboxymethylcellulose sodium, and the like. Examples of the preservative include methyl p-hydroxybenzoate, ethyl p-hydroxybenzoate, benzyl alcohol, chlorobutanol, and the like.
Although osmotic pressure of the aqueous solution composition of the present invention is not particularly limited, the pressure is usually 200 to 700 mOsm/kg, preferably 200 to 600 mOsm/kg, and osmotic pressure ratio to that of physiological saline is preferably 0.6 to 3, especially 0.6 to 2.

An exemplary preferred embodiment of the aqueous solution composition of the present invention is, for example, an aqueous solution composition containing (S)-1-(4-chloro-5-isoquinolinesulfonyl)-3-(methylamino)pyrrolidine monohydrochloride as an active ingredient but not containing citric acid or a salt thereof, and further containing a pH adjustor other than citric acid or a salt thereof, and having a pH of 5 to 9. In this aqueous solution composition, after six-month storage at 40°C or two-month storage at 80°C, purity of (S)-1-(4-chloro-5-isoquinolinesulfonyl)-3-(methylamino)pyrrolidine is 98% or higher, and therefore the composition has sufficient stability as an eye drop. In a physiological saline solution of (S)-1-(4-chloro-5-isoquinolinesulfonyl)-3-(methylamino)pyrrolidine, an increase in pH with passage of time is observed. However, in the aqueous solution composition of the present invention containing a pH adjustor, an increase in pH is suppressed. Long-term storage stability and pH maintenance effect of the aqueous solution composition of the present invention containing (S)-1-(4-chloro-5-isoquinolinesulfonyl)-3-aminopyrrolidine monohydrochloride can also be confirmed by performing a similar test.

As the aqueous solution composition of the present invention, aqueous solutions of (S)-1-(4-chloro-5-isoquinolinesulfonyl)-3-(methylamino)pyrrolidine of which pH was adjusted to 4, 5, 6, 7, 8 or 9 were prepared, and administered to normal rabbits by instillation, and intraocular pressure of the rabbits was measured over time. As a result, the intraocular pressure reducing action in the rabbits became higher in a pH-dependent manner, i.e., a higher pH achieved a higher intraocular pressure reducing action. This tendency can also be confirmed for an (S)-1-(4-chloro-5-isoquinolinesulfonyl)-3-aminopyrrolidine aqueous solution by performing the same test.

The aqueous solution composition of the present invention can exhibit superior intraocular pressure reducing action even in a patient having normal intraocular pressure. Therefore, a medicament containing the aqueous solution composition of the present invention is useful as a medicament for prophylactic and/or therapeutic treatment of glaucoma, a medicament for prophylactic and/or therapeutic treatment of ocular hypertension, and the like. Examples of glaucoma include, for example, primary open-angle glaucoma, normal ocular pressure glaucoma, hypersecretion glaucoma, ocular hypertension, acute angle-closure glaucoma, chronic angle-closure glaucoma, plateauiris syndrome, mixed glaucoma, steroid-induced glaucoma, glaucoma capsulare, pigmentary glaucoma, amyloid glaucoma, neovascular glaucoma, malignant glaucoma, and the like.

Although a route of administration of the aqueous solution composition of the present invention is not particularly limited, a particularly preferred example of administration route includes instillation. Therefore, an eye drop preparation containing the aqueous solution composition of the present invention is one of preferred embodiments of the present invention. The eye drop is preferably prepared as a sterile preparation, and can be prepared as a sterile preparation by performing sterilization and/or sterile filtration and the like in a conventional manner.

As a container in which the aqueous solution composition of the present invention is filled, a container having a permeability of 40% or lower for ultraviolet lights having a wavelength of 350 nm or shorter is preferred, and a container having such a permeability of 30% or lower is more preferred.
As a packaging container in which a container filled with the aqueous solution composition of the present invention is stored, a packaging container having a permeability of 20% or lower for ultraviolet lights having a wavelength of 350 nm or shorter is preferred, and a packaging container having a permeability of 10% or lower for ultraviolet lights having a wavelength of 350 nm or shorter is more preferred. The permeability is highly preferably 5% or lower, most preferably 1% or lower. Although shape of the packaging container is not particularly limited, preferred examples include box, bag, and the like.
An embodiment in which the aqueous solution composition of the present invention is filled in a container having a permeability of 40% or lower, preferably 30% or lower, for ultraviolet lights having a wavelength of 350 nm or shorter, and the container is stored in a packaging container having a permeability of 20% or lower, preferably 10% or lower, for ultraviolet lights having a wavelength of 50 nm or shorter is a particularly preferred embodiment of the present invention.

### Examples

The present invention will be more specifically explained with reference to examples. However, the scope of the present invention is not limited to the following examples.

### [Example 1] Preparation of (S)-1-(4-chloro-5-isoquinolinesulfonyl)-3-(methylamino)pyrrolidine monohydrochloride

According to the method described in International Patent Publication WO2007/026664, (S)-1-(4-chloro-5-isoquinohnesulfonyl)-3-(methylamino)pyrrolidine was obtained as hydrochloride (1.50 g), and this salt was dissolved in water (52 mL). The resulting solution was vigorously stirred, and slowly added dropwise with 2 mol/L aqueous sodium hydroxide (4.13 mL, Wako Pure Chemical Industries Co., Ltd.) under ice cooling. The resulting suspension was further stirred at room temperature for 1 hour, and then added with methylene chloride (30 mL), and the organic layer was separated. Further, the aqueous layer was extracted with methylene chloride (30 mL), and the combined organic layer was washed with water (50 mL), and dried over anhydrous magnesium sulfate. The solvent was evaporated under reduced pressure, and the residue was added with ethyl acetate (10 mL) and n-hexane (20 mL). The deposited solid was collected by filtration, and dried by heating at 50°C for 20 hours under reduced pressure to obtain (S)-1-(4-chloro-5-isoquinolinesulfonyl)-3-(methylamino)pyrrolidine (845 mg)). Further, the solvent of the filtrate was evaporated under reduced pressure, and the residue was added with ethyl acetate (3 mL) and n-hexane (6 mL). The deposited solid was collected by filtration, and dried overnight by heating at 50°C under reduced pressure to obtain (S)-1-(4-chloro-5-isoquinolinesulfonyl)-3-(methylamino)pyrrolidine (195 mg).

(S)-1-(4-Chloro-5-isoquinolinesulfonyl)-3-(methylamino)pyrrolidine (1.04 g) was dissolved in ethanol (25 mL)). This solution was added dropwise with 5 mol/L hydrochloric acid (0.606 mL) at room temperature. The mixture was further stirred at room temperature for 8 hours and 30 minutes, and then added with ethanol (20 mL)). The deposited solid was collected by filtration, washed with ethanol, and then dried overnight by heating at 60°C under reduced pressure to obtain the title compound (1.06 g).

When number of chloride salt of the compound obtained in Example 1 was confirmed by ion exchange chromatography under the conditions described below, 1.0 of chloride ion was detected per one molecule of (S)-1-(4-chloro-5-isoquinolinesulfonyl)-3-(methylamino)pyrrolidine, and thus it was confirmed that this substance was a salt consisting of (S)-1-(4-chloro-5-isoquinolinesulfonyl)-3-(methylamino)pyrrolidine added with one hydrochloric acid molecule.

[Example 2] Preparation of (S)-1-(4-chloro-5-isoquinolinesulfonyl)-3-(methylamino)pyrrolidine monohydrochloride aqueous solutions not containing citric acid or a salt thereof

### (1) Physiological saline solution

Sodium chloride (18 g) was dissolved in distilled water (2 L), and this solution was used as a solvent. (S)-1-(4-Chloro-5-isoquinolinesulfonyl)-3-(methylamino)-pyrrolidine monohydrochloride (12 mg) was added with the solvent (60 mL) and dissolved in the solvent, and pH of the solution was measured.

### (2) 10 mM Phosphate buffer (pH 6) solution

Sodium dihydrogen phosphate dihydrate (0.78 g) was dissolved in the solvent of Example 2, (1) (500 mL). This solution (50 mL) was added with a solution of disodium hydrogen phosphate dodecahydrate (1.432 g) dissolved in the solvent of Example 2, (1) (400 mL), and the mixture was adjusted to pH 6.0 to afford a 10 mM phosphate buffer (pH 6) solvent. (S)-1-(4-Chloro-5-isoquinolinesulfonyl)-3-(methylamino)pyrrolidine monohydrochloride (12 mg) was added with the 10 mM phosphate buffer (pH 6) solvent (60 mL) and dissolved in the solvent, and it was confirmed that pH of the solution was around 6. This solution was regarded as an about 0.55 mM (S)-1-(4-chloro-5-isoquinolinesulfonyl)-3-(methylamino)pyrrolidine monohydrochloride solution in the 10 mM phosphate buffer (pH 6) solvent.

### (3) 10 mM Phosphate buffer (pH 7) solution

Sodium dihydrogen phosphate dihydrate (0.78 g) was dissolved in the solvent of Example 2, (1) (500 mL). This solution (50 mL) was added with a solution of disodium hydrogen phosphate dodecahydrate (1.432 g) dissolved in the solvent of Example 2, (1) (400 mL), and the mixture was adjusted to pH 7.0 to afford a 10 mM phosphate buffer (pH 7) solvent. (S)-1-(4-Chloro-5-isoquinolinesulfonyl)-3-(methylamino)pyrrolidine monohydrochloride (12 mg) was added with the 10 mM phosphate buffer (pH 7) solvent (60 mL) and dissolved in the solvent, and it was confirmed that pH of the solution was around 7. This solution was regarded as an about 0.55 mM (S)-1-(4-chloro-5-isoquinolinesulfonyl)-3-(methylamino)pyrrolidine monohydrochloride solution in the 10 mM phosphate buffer (pH 7) solvent.

### (4) 10 mM Phosphate buffer (pH 8) solution

Sodium dihydrogen phosphate dihydrate (0.78 g) was dissolved in the solvent of Example 2, (1) (500 mL). This solution (50 mL) was added with a solution of disodium hydrogen phosphate dodecahydrate (1.432 g) dissolved in the solvent of Example 2, (1) (400 mL), and the mixture was adjusted to pH 8.0 to afford a 10 mM phosphate buffer (pH 8) solvent. (S)-1-(4-Chloro-5-isoquinolinesulfonyl)-3-(methylamino)pyrrolidine monohydrochloride (12 mg) was added with the 10 mM phosphate buffer (pH 8) solvent (60 mL) and dissolved in the solvent, and it was confirmed that pH of the solution was around 8. This solution was regarded as an about 0.55 mM (S)-1-(4-chloro-5-isoquinolinesulfonyl)-3-(methylamino)pyrrolidine monohydrochloride solution in the 10 mM phosphate buffer (pH 8) solvent.

### (5) 10 mM Phosphate buffer (pH 5) solution

Sodium dihydrogen phosphate dihydrate (0.78 g) was dissolved in the solvent of Example 2, (1) (500 mL). This solution (50 mL) was added with a solution of disodium hydrogen phosphate dodecahydrate (1.43 g) dissolved in the solvent of Example 2, (1) (400 mL), and the mixture was adjusted to pH 5.0 to afford a 10 mM phosphate buffer (pH 5) solvent. (S)-1-(4-Chloro-5-isoquinolinesulfonyl)-3-(methylamino)pyrrolidine monohydrochloride (10 mg) was added with the 10 mM phosphate buffer (pH 5) solvent (50 mL) and dissolved in the solvent, and it was confirmed that pH of the solution was around 5. This solution was regarded as an about 0.55 mM (S)-1-(4-chloro-5-isoquinolinesulfonyl)-3-(methylamino)pyrrolidine monohydrochloride solution in the 10 mM phosphate buffer (pH 5) solvent.

### (6) 10 mM Acetate buffer (pH 5) solution

Sodium acetate trihydrate (0.48 g) was dissolved in the solvent of Example 2, (1) (500 mL). This solution (50 mL) was added with a solution of acetic acid (100) (0.30 g) dissolved in the solvent of Example 2, (1) (500 mL), and the mixture was adjusted to pH 5.0 to afford a 10 mM acetate buffer (pH 5) solvent. (S)-1-(4-Chloro-5-isoquinolinesulfonyl)-3-(methylamino)pyrrolidine monohydrochloride (10 mg) was added with the 10 mM acetate buffer (pH 5) solvent (50 mL) and dissolved in the solvent, and it was confirmed that pH of the solution was around 5. This solution was regarded as an about 0.55 mM (S)-1-(4-chloro-5-isoquinolinesulfonyl)-3-(methylamino)pyrrolidine monohydrochloride solution in the 10 mM acetate buffer (pH 5) solvent.

### (7) 10 mM Borate buffer (pH 8) solution

Boric acid (0.31 g) was dissolved in the solvent of Example 2, (1) (500 mL). This solution (50 mL) was added with a solution of sodium tetraborate decahydrate (0.48 g) dissolved in the solvent of Example 2, (1) (500 mL), and the mixture was adjusted to pH 8.0 to afford a 10 mM borate buffer (pH 8) solvent. (S)-1-(4-Chloro-5-isoquinolinesulfonyl)-3-(methylamino)pyrrolidine monohydrochloride (10 mg) was added with the 10 mM borate buffer (pH 8) solvent (50 mL) and dissolved in the solvent, and it was confirmed that pH of the solution was around 8. This solution was regarded as an about 0.55 mM (S)-1-(4-chloro-5-isoquinolinesulfonyl)-3-(methylamino)pyrrolidine monohydrochloride solution in the 10 mM borate buffer (pH 8) solvent.

### (8) 10 mM Borate buffer (pH 9) solution

Sodium tetraborate decahydrate (0.48 g) was dissolved in the solvent of Example 2, (1) (500 mL). This solution (50 mL) was added with a solution of boric acid (0.31 g) dissolved in the solvent of Example 2, (1) (500 mL), and the mixture was adjusted to pH 9.0 to afford a 10 mM borate buffer (pH 9) solvent. (S)-1-(4-Chloro-5-isoquinolinesulfonyl)-3-(methylamino)pyrrolidine monohydrochloride (10 mg) was added with the 10 mM borate buffer (pH 9) solvent (50 mL) and dissolved in the solvent, and it was confirmed that pH of the solution was around 9. This solution was regarded as an about 0.55 mM (S)-1-(4-chloro-5-isoquinolinesulfonyl)-3-(methylamino)pyrrolidine monohydrochloride solution in the 10 mM borate buffer (pH 9) solvent.

### (9) 10 mM Tris(hydroxymethyl)aminomethane buffer (pH 8) solution

Tris(hydroxymethyl)aminomethane (0.12 g) was dissolved in the solvent of Example 2, (1) (80 mL). This solution was added with a solution prepared by adding the solvent of Example 2, (1) to accurately measured 2 mL of 2 mol/L hydrochloric acid to obtain a volume of exactly 20 mL, the mixture was adjusted to pH 8.0, and then added with the solvent of Example 2, (1) up to a volume of 100 mL to afford a 10 mM tris(hydroxymethyl)aminomethane buffer (pH 8) solvent. (S)-1-(4-Chloro-5-isoquinolinesulfonyl)-3-(methylamino)pyrrolidine monohydrochloride (10 mg) was added with the 10 mM tris(hydroxymethyl)aminomethane buffer (pH 8) solvent (50 mL) and dissolved in the solvent, and it was confirmed that pH of the solution was around 8. This solution was regarded as an about 0.55 mM (S)-1-(4-chloro-5-isoquinolinesulfonyl)-3-(methylamino)pyrrolidine monohydrochloride solution in the 10 mM tris(hydroxymethyl)aminomethane buffer (pH 8) solvent.

[Comparative Example 1] Preparation of (S)-1-(4-chloro-5-isoquinolinesulfonyl)-3-(methylamino)pyrrolidine monohydrochloride aqueous solutions containing citric acid or a salt thereof

### (1) 10 mM Citrate buffer (pH 4) solution

Disodium hydrogen citrate 1.5 hydrate (1.432 g) was dissolved in the solvent of Example 2, (1) (400 mL). This solution (50 mL) was added with a solution of citric acid monohydrate (0.84 g) dissolved in the solvent of Example 2, (1) (400 mL), and the mixture was adjusted to pH 4.0 to afford a 10 mM citrate buffer (pH 4) solvent. (S)-1-(4-Chloro-5-isoquinolinesulfonyl)-3-(methylamino)pyrrolidine monohydrochloride (12 mg) was added with the 10 mM citrate buffer (pH 4) solvent (60 mL) and dissolved in the solvent, and it was confirmed that pH of the solution was around 4. This solution was regarded as an about 0.55 mM (S)-1-(4-chloro-5-isoquinolinesulfonyl)-3-(methylamino)pyrrolidine monohydrochloride solution in the 10 mM citrate buffer (pH 4) solvent.

### (2) 10 mM Citrate buffer (pH 5) solution

Disodium hydrogen citrate 1.5 hydrate (1.432 g) was dissolved in the solvent of Example 2, (1) (400 mL). This solution (50 mL) was added with a solution of citric acid monohydrate (0.84 g) dissolved in the solvent of Example 2, (1) (400 mL), and the mixture was adjusted to pH 5.0 to afford a 10 mM citrate buffer (pH 5) solvent. (S)-1-(4-Chloro-5-isoquinolinesulfonyl)-3-(methylamino)pyrrolidine monohydrochloride (12 mg) was added with the 10 mM citrate buffer (pH 5) solvent (60 mL) and dissolved in the solvent, and it was confirmed that pH of the solution was around 5. This solution was regarded as an about 0.55 mM (S)-1-(4-chloro-5-isoquinolinesulfonyl)-3-(methylamino)pyrrolidine monohydrochloride solution in the 10 mM citrate buffer (pH 5) solvent.

### (3) 10 mM Citrate phosphate buffer (pH 6) solution

Sodium dihydrogen phosphate dihydrate (0.78 g) and citric acid monohydrate (1.05 g) were dissolved in the solvent of Example 2, (1) (500 mL). This solution (50 mL) was added with a solution of disodium hydrogen phosphate dodecahydrate (1.43 g) and sodium citrate dihydrate (1.18 g) dissolved in the solvent of Example 2, (1) (400 mL), and the mixture was adjusted to pH 6.0 to afford a 10 mM citrate phosphate buffer (pH 6) solvent. (S)-1-(4-Chloro-5-isoquinolinesulfonyl)-3-(methylamino)pyrrolidine monohydrochloride (10 mg) was added with the 10 mM citrate phosphate buffer (pH 6) solvent (50 mL) and dissolved in the solvent, and it was confirmed that pH of the solution was around 6. This solution was regarded as an about 0.55 mM (S)-1-(4-chloro-5-isoquinolinesulfonyl)-3-(methylamino)pyrrolidine monohydrochloride solution in the 10 mM citrate phosphate buffer (pH 6) solvent.

### [Test Example 1] Stability test of (S)-1-(4-chloro-5-isoquinolinesulfonyl)-3-(methylamino)pyrrolidine monohydrochloride aqueous solutions (1)

In the following tests, IN801 (Yamato Scientific Co., Ltd.) was used as an incubator at 40°C, and IS600 (Yamato Scientific Co., Ltd.) was used as an incubator at 50 to 80°C.
The solutions of Example 2, (1) to (4) and Comparative Example 1, (1) and (2) were filtered through a membrane filter having a pore size of 0.22 µm, each filled in 18 brown glass ampoules in a volume of 2 mL each, and the ampoules were sealed by melting. These samples were stored at 40 to 80°C for each period of time shown in Table 1.

**[Table 1]**

| Storage condition | Storage period of time |
|---|---|
| 40°C | 1 month, 2 months, 6 months |
| 50°C | 1 month, 2 months, 6 months |
| 60°C | 2 weeks, 4 weeks, 2 months |
| 70°C | 2 weeks, 4 weeks, 2 months |
| 80°C | 1 week, 2 weeks, 2 months |

### <Measurement method>

After pH values of the solutions of Example 2, (1) to (4) and Comparative Example 1, (1) and (2) were measured before and after the storage, purity of (S)-1-(4-chloro-5-isoquinolinesulfonyl)-3-(methylamino)pyrrolidine was measured by high performance liquid chromatography under the following conditions. The (S)-1-(4-chloro-5-isoquinolinesulfonyl)-3-(methylamino)pyrrolidine purity was calculated by the area percentage method.
Conditions for pH measurement
pH Meter: HM-30G (DKK-TOA Corporation)
Measurement temperature: room temperature

### HPLC conditions

Liquid chromatography apparatus: LC-10A Series HPLC system (Shimadzu Corporation), or Agilent 1100 Series HPLC system (Agilent Technologies Inc.)
Injection volume: 10 µL
Detector: Ultraviolet absorptiometer (measurement wavelength: 245 nm)
Column: XBridge Shield RP18 5 µm, internal diameter: 4.6 mm, length: 15 cm
(Waters Corporation)
Column temperature: constant temperature around 40°C
Mobile phase A: 20 mmol/L sodium phosphate buffer (pH 7.0)
Mobile phase B: methanol
Liquid feeding program: Concentration gradient controlled by changing the mixing ratio of the mobile phase A and the mobile phase B as shown in Table 2

**[Table 2]**

| Time after injection (minute) | Mobile phase A (%) | Mobile phase B (%) |
|---|---|---|
| 0 to 45.5 | 80→15 | 20→85 |
| 45.5 to 54 | 15 | 85 |
| 54 to 69 | 80 | 20 |

Flow rate: 1.0 mL/minute
Syringe washing solution methanol/water (4:1) mixture
(S)-1-(4-Chloro-5-isoquinolinesulfonyl)-3-(methylamino)pyrrolidine was eluted at about 12 minutes under the conditions of this test.

### <Results>

In the physiological saline solution of (S)-1-(4-chloro-5-isoquinohnesulfonyl)-3-(methylamino)pyrrolidine monohydrochloride (Example 2, (1)), decrease in the purity with passage of time was not observed, whist elevation of pH with passage of time was observed under the following conditions.
Among the aqueous solutions containing (S)-1-(4-chloro-5-isoquinoline-sulfonyl)-3-(methylamino)pyrrolidine monohydrochloride and added with phosphoric acid or a salt thereof as a pH adjustor (Example 2, (2) to (4)), the solution of pH 6 ((4) mentioned above) was most stable, and the solutions of pH 7 ((5) mentioned above) and pH 8 ((6) mentioned above) became less stable in this order. However, even in the solution of pH 8, the (S)-1-(4-chloro-5-isoquinolinesulfonyl)-3-(methylamino)pyrrolidine purity was 99% or higher at least after the storage at 40°C for 6 months.

It was also found that, in the aqueous solutions containing (S)-1-(4-chloro-5-isoquinolinesulfonyl)-3-(methylamino)pyrrolidine monohydrochloride at pH 4 (Comparative Example 1, (1)) and pH 5 (Comparative Example 1, (2)) and added with citric acid or a salt thereof as a pH adjustor, stability of (S)-1-(4-chloro-5-isoquinolinesulfonyl)-3-(methylamino)pyrrolidine monohydrochloride was inferior to that observed in the aqueous solutions added with phosphoric acid or a salt thereof.
In the aqueous solutions of (S)-1-(4-chloro-5-isoquinohnesulfonyl)-3-(methylamino)pyrrolidine monohydrochloride added with a pH adjustor (Example 2, (2) to (4), Comparative Example 1 (1), and (2)), significant change of pH was not observed under all the conditions.
The results are shown in Table 3. In Table 3, only the results at the time of the starts and ends of the tests under the respective temperature conditions are summarized.

**[Table 3]**

| Solution | Item | Start | 40°C 6months | 50°C 6months | 60°C 2months | 70°C 2months | 80°C 2months |
|---|---|---|---|---|---|---|---|
| Example 2, (1) | pH | 5.98 | 6.28 | 6.60 | 6.50 | 6.92 | 7.01 |
| Physiological saline | Purity (%) | 98.63 | 99.79 | 99.74 | 99.74 | 99.27 | 98.85 |
| Example 2, (2) | pH | 6.00 | 6.04 | 6.10 | 6.06 | 6.10 | 6.13 |
| Phosphate buffer (pH 6) | Purity (%) | 99.23 | 99.77 | 99.72 | 99.62 | 99.42 | 98.82 |
| Example 2, (3) | pH | 7.01 | 7.01 | 7.03 | 7.02 | 7.02 | 7.03 |
| Phosphate buffer (pH 7) | Purity (%) | 99.35 | 99.79 | 99.66 | 99.66 | 98.91 | 97.71 |
| Example 2, (4) | pH | 7.79 | 7.82 | 7.83 | 7.81 | 7.83 | 7.83 |
| Phosphate buffer (pH 8) | Purity (%) | 99.40 | 99.74 | 99.24 | 99.48 | 98.58 | 97.06 |
| Comparative Example 1, (1) | pH | 4.02 | 4.01 | 4.02 | 4.03 | 4.06 | 4.11 |
| Citrate buffer (pH 4) | Purity (%) | 97.26 | 97.59 | 96.54 | 96.87 | 94.43 | 90.08 |
| Comparative Example 1, (2) | pH | 5.00 | 5.02 | 5.08 | 5.03 | 5.20 | 5.61 |
| Citrate buffer (pH 5) | Purity (%) | 98.05 | 96.80 | 94.12 | 94.51 | 88.78 | 78.17 |

### [Test Example 2] Stability test of (S)-1-(4-chloro-5-isoquinolinesulfonyl)-3-(methylamino)pyrrolidine monohydrochloride aqueous solutions (2)

The solutions of Example 2, (5) to (8) and Comparative Example 1, (1) were filtered through a membrane filter having a pore size of 0.22 µm, each filled in 5 brown glass ampoules in a volume of 2 mL each, and the ampoules were sealed by melting. Further, the solution of Example 2, (9) was filtered through a membrane filter having a pore size of 0.22 µm, and filled in 3 brown glass ampoules in a volume of 2 mL each, and the ampoules were sealed with stoppers. The solutions of Example 2, (5) to (8) and Comparative Example 1, (3) were stored at 80°C for 2 months, and the solution of Example 2, (9) was stored at 80°C for 2 weeks. The measurement was performed in the same manner as that of Test Example 1.

### <Results>

In the solution of Comparative Example 1, (3) containing citric acid or a salt thereof, decrease of the purity was observed after the storage at 80°C for 2 months even at pH 6. Whilst in the solutions of Example 2, (5), (6), (7) and (8) not containing citric acid or a salt thereof, significant decrease of the purity was not observed even after the storage at 80°C for 2 months. Further, although the solution of Example 2, (9) was stored at 80°C only for 2 weeks, the solution was considered not to give significant decrease in the purity after storage at 80°C for 2 months, judging from the change of the purity observed after 2 weeks. The results are shown in Table 4.

**[Table 4]**

| Solution | Test | Initial value | 80°C 2 weeks | 80°C 1 month | 80°C 2 months |
|---|---|---|---|---|---|
| Example 2, (5) | pH | 5.06 | 5.34 | 5.48 | 5.64 |
| Phosphate buffer (pH 5) | Purity (%) | 100.00 | 99.82 | 99.71 | 99.51 |
| Example 2, (6) | pH | 4.99 | 5.04 | 5.05 | 5.08 |
| Acetate buffer (pH 5) | Purity (%) | 100.00 | 99.88 | 99.76 | 99.52 |
| Example 2, (7) | pH | 7.78 | 7.82 | 7.84 | 7.83 |
| Borate buffer (pH 8) | Purity (%) | 100.00 | 99.51 | 98.97 | 98.37 |
| Example 2, (8) | pH | 8.84 | 8.84 | 8.85 | 8.85 |
| Borate buffer (pH 9) | Purity (%) | 100.00 | 99.76 | 99.25 | 98.61 |
| Example 2, (9) | pH | 7.92 | 7.98 | - | - |
| Tris buffer (pH 8)* | Purity (%) | 100.00 | 99.67 | - | - |
| Comparative Example 1, (3) | pH | 6.01 | 6.20 | 6.38 | 6.62 |
| Citrate phosphate buffer (pH 6) | Purity (%) | 98.89 | 88.27 | 82.93 | 78.35 |

| | | | | | |
|---|---|---|---|---|---|
| -: The solution of Example 2, (9) was stored at 80°C only for 2 weeks. *: Tris(hydroxymethyl)aminomethane buffer | | | | | |

From the results of Test Examples 1 and 2, it was found that, by dissolving (S)-1-(4-chloro-5-isoquinolinesulfonyl)-3-(methylamino)pyrrolidine or a pharmacologically acceptable salt thereof in a solvent not containing citric acid or a salt thereof and adjusted to pH 5 to 9 using phosphoric acid or a salt thereof, acetic acid or a salt thereof, tris(hydroxymethyl)aminomethane or a salt thereof, or boric acid or a salt thereof as a pH adjustor (containing 0.9% sodium chloride as an isotonic agent), a stable aqueous solution containing (S)-1-(4-chloro-5-isoquinolinesulfonyl)-3-(methylamino)pyrrolidine or a pharmacologically acceptable salt thereof was successfully obtained.

### [Example 3] Preparation of (S)-1-(4-chloro-5-isoquinolinesulfonyl)-3-aminopyrrolidine monohydrochloride

(S)-1-(4-Chloro-5-isoquinolinesulfonyl)-3-aminopyrrolidine hydrochloride (5.500 g) obtained according to the method described in International Patent Publication WO2007/026664 was dissolved in water (50 mL). This solution was vigorously stirred, and slowly added dropwise with 1 mol/L aqueous sodium hydroxide (29.32 mL, Wako Pure Chemical Industries Co., Ltd.) under ice cooling. The mixture was added with ethyl acetate (300 mL) and water (20 mL), and the organic layer was evaporated. Further, the aqueous layer was extracted with methylene chloride, and the combined the organic layer was washed with water, and dried over anhydrous magnesium sulfate. The solvent was evaporated under reduced pressure, and the residue was added with ethyl acetate (35 mL) and n-hexane (105 mL). The deposited solid was collected by filtration, washed with a mixture of n-hexane/ethyl acetate (4:1), and then vacuum dried at 50°C under reduced pressure to obtain (S)-1-(4-chloro-5-isoquinolinesulfonyl)-3-aminopyrrolidine (4.24 g).

(S)-1-(4-Chloro-5-isoquinolinesulfonyl)-3-aminopyrrolidine (1.30 g) was dissolved in ethanol (52 mL). This solution was added dropwise with 5 mol/L hydrochloric acid (0.7922 mL) at room temperature. The mixture was further stirred overnight at room temperature, and then the deposited solid was collected by filtration, washed with ethanol and dichloromethane, and then dried by heating at 60°C under reduced pressure to obtain the title compound.
When number of chloride salt of the compound obtained in Example 3 was confirmed by ion exchange chromatography under the conditions described below, 1.0 of chloride ion was detected per one molecule of (S)-1-(4-chloro-5-isoquinolinesulfonyl)-3-aminopyrrolidine, and thus it was confirmed that this substance was a salt consisting of (S)-1-(4-chloro-5-isoquinolinesulfonyl)-3-aminopyrrolidine added with one hydrochloric acid molecule.

### [Example 4] Preparation of (S)-1-(4-chloro-5-isoquinolinesulfonyl)-3-aminopyrrolidine monohydrochloride aqueous solutions not containing citric acid or a salt thereof (1) Physiological saline solution

(S)-1-(4-Chloro-5-isoquinolinesulfonyl)-3-aminopyrrolidine monohydrochloride (12 mg) was added with the solvent of Example 2, (1) (60 mL) and dissolved in the solvent, and pH of the solution was measured.

### (2) 10 mM Phosphate buffer (pH 6) solution

(S)-1-(4-Chloro-5-isoquinolinesulfonyl)-3-aminopyrrolidine monohydrochloride (12 mg) was added with the 10 mM phosphate buffer (pH 6) solvent of Example 2, (2) (60 mL) and dissolved in the solvent, and it was confirmed that pH of the solution was around 6. This solution was regarded as an about 0.55 mM (S)-1-(4-chloro-5-isoquinolinesulfonyl)-3-aminopyrrolidine monohydrochloride solution in the 10 mM phosphate buffer (pH 6) solvent.

### (3) 10 mM Phosphate buffer (pH 7) solution

(S)-1-(4-Chloro-5-isoquinolinesulfonyl)-3-aminopyrrolidine monohydrochloride (12 mg) was added with the 10 mM phosphate buffer (pH 7) solvent of Example 2, (3) (60 mL) and dissolved in the solvent, and it was confirmed that pH of the solution was around 7. This solution was regarded as an about 0.55 mM (S)-1-(4-chloro-5-isoquinolinesulfonyl)-3-aminopyrrolidine monohydrochloridesolution in the 10 mM phosphate buffer (pH 7) solvent.

### (4) 10 mM Phosphate buffer (pH 8) solution

(S)-1-(4-Chloro-5-isoquinolinesulfonyl)-3-aminopyrrolidine monohydrochloride (12 mg) was added with the 10 mM phosphoric acid buffer (pH 8) solvent of Example 2, (4) (60 mL) and dissolved in the solvent, and it was confirmed that pH of the solution was around 8. This solution was regarded as an about 0.55 mM (S)-1-(4-chloro-5-isoquinolinesulfonyl)-3-aminopyrrolidine monohydrochloride solution in the 10 mM phosphoric acid buffer (pH 8) solvent.

### [Comparative Example 2] Preparation of (S)-1-(4-chloro-5-isoquinolinesulfonyl)-3-aminopyrrolidine monohydrochloride aqueous solutions containing citric acid or a salt thereof

### (1) 10 mM Citrate buffer (pH 4) solution

(S)-1-(4-Chloro-5-isoquinolinesulfonyl)-3-aminopyrrolidine monohydrochloride (12 mg) was added with the 10 mM citrate buffer (pH 4) solvent of Comparative Example 2, (1) (60 mL) and dissolved in the solvent, and it was confirmed that pH of the solution was around 4. This solution was regarded as an about 0.55 mM (S)-1-(4-chloro-5-isoquinolinesulfonyl)-3-aminopyrrolidine monohydrochloride solution in the 10 mM citrate buffer (pH 4) solvent.

### (2) 10 mM Citrate buffer (pH 5) solution

(S)-1-(4-Chloro-5-isoquinolinesulfonyl)-3-aminopyrrolidine monohydrochloride (12 mg) was added with the 10 mM citrate buffer (pH 5) solvent of Comparative Example 1, (2) (60 mL) and dissolved in the solvent, and it was confirmed that pH of the solution was around 5. This solution was regarded as an about 0.55 mM (S)-1-(4-chloro-5-isoquinolinesulfonyl)-3-aminopyrrolidine monohydrochloride solution in the 10 mM citrate buffer (pH 5) solvent.

### [Test Example 3]

### Stability test of (S)-1-(4-chloro-5-isoquinolinesulfonyl)-3-aminopyrrolidine monohydrochloride aqueous solutions

The same experiment as that of Test Example 1 was performed by using (S)-1-(4-chloro-5-isoquinolinesulfonyl)-3-aminopyrrolidine monohydrochloride instead of (S)-1-(4-chloro-5-isoquinolinesulfonyl)-3-(methylamino)pyrrolidine monohydrochloride.
In the solutions at pH 4 and pH 5 using citric acid or a salt thereof as a pH adjustor (solutions of Comparative Example 2, (1) and (2)), decrease of the purity was observed as in the case of (S)-1-(4-chloro-5-isoquinolinesulfonyl)-3-(methylamino)pyrrolidine monohydrochloride. Whilst it was confirmed that where the substance was dissolved in a solvent adjusted to pH 6 to 8 by using phosphoric acid or a salt thereof as a pH adjustor (containing 0.9% sodium chloride as an isotonic agent), the substance was stable for 4 weeks at 60 to 70°C, or for 2 weeks at 80°C (solutions of Example 4, (1) to (4)).
The results are shown in Table 5.

**[Table 5]**

| Solution | Item | Start | 60°C 4 weeks | 70°C 4 weeks | 80°C 2 weeks |
|---|---|---|---|---|---|
| Example 4, (1) | pH | 5.34 | 6.39 | 6.53 | 6.66 |
| Physiological saline | Purity (%) | 99.44 | 98.89 | 99.39 | 99.05 |
| Example 4, (2) | pH | 6.02 | 6.07 | 6.07 | 6.04 |
| Phosphate buffer (pH 6) | Purity (%) | 99.61 | 99.31 | 99.49 | 99.36 |
| Example 4, (3) | pH | 6.99 | 7.03 | 7.06 | 7.01 |
| Phosphate buffer (pH 7) | Purity (%) | 99.53 | 99.44 | 99.41 | 99.20 |
| Example 4, (4) | pH | 7.77 | 7.83 | 7.83 | 7.83 |
| Phosphate buffer (pH 8) | Purity (%) | 99.66 | 99.38 | 99.23 | 99.17 |
| Comparative Example 2, (1) | pH | 4.02 | 4.03 | 4.01 | 4.03 |
| Citrate buffer (pH 4) | Purity (%) | 97.90 | 94.98 | 92.57 | 91.32 |
| Comparative Example 2, (2) | pH | 5.00 | 5.02 | 5.09 | 5.13 |
| Citrate buffer (pH 5) | Purity (%) | 98.49 | 91.60 | 83.34 | 78.43 |

From the results of Test Example 3, it was found that where (S)-1-(4-chloro-5-isoquinolinesulfonyl)-3-aminopyrrolidine or a pharmacologically acceptable salt thereof was dissolved in a solvent adjusted to pH 5 to 8 by using phosphoric acid or a salt thereof as a pH adjustor and not containing citric acid or a salt thereof (containing 0.9% sodium chloride as an isotonic agent), a stable aqueous solution containing (S)-1-(4-chloro-5-isoquinolinesulfonyl)-3-aminopyrrolidine or a pharmacologically acceptable salt thereof was successfully obtained. Further, although tests were not performed, it is estimated that by dissolving (S)-1-(4-chloro-5-isoquinolinesulfonyl)-3-aminopyrrolidine or a pharmacologically acceptable salt thereof in a solvent not containing citric acid or a salt thereof and adjusted to pH 5 to 9 using phosphoric acid or a salt thereof, acetic acid or a salt thereof, tris(hydroxymethyl)aminomethane or a salt thereof, or boric acid or a salt thereof (containing 0.9% sodium chloride as an isotonic agent), a stable aqueous solution containing (S)-1-(4-chloro-5-isoquinolinesulfonyl)-3-aminopyrrolidine or a pharmacologically acceptable salt thereof can also be obtained.

### [Test Example 4] Rabbit intraocular pressure reduction test

In order to examine difference in the intraocular pressure reducing effect depending on a different pH of (S)-1-(4-chloro-5-isoquinolinesulfonyl)-3-(methylamino)pyrrolidine solution, the following test was performed by using Japanese white rabbits as experimental animals. 1. Preparation of aqueous solutions of test compound

### Preparation of (S)-1-(4-chloro-5-isoquinolinesulfonyl)-3-(methylamino)pyrrolidine solutions

(S)-1-(4-Chloro-5-isoquinolinesulfonyl)-3-(methylamino)pyrrolidine hydrochloride obtained by the method described in International Patent Publication WO2007/026664 was dissolved in physiological saline, the solution was adjusted to pH 4, 5, 6, 7, 8 and 9 by adding sodium hydroxide, and the final concentration was adjusted to 0.5 mM. These solutions were used as (S)-1-(4-chloro-5-isoquinolinesulfonyl)-3-(methylamino)pyrrolidine solutions having different pH values.

### 2. Test method

The rabbits used were sufficiently conditioned for ophathalmic tonometry test, and used as groups each consisting of 6 animals. After a Benoxil ophthalmic solution 0.4% (an ophthalmologic local anesthesant, Santen Pharmaceutical Co., Ltd.) was applied to both eyes of each rabbit, intraocular pressure value before instillation was measured (initial intraocular pressure value, both right and left eyes) by using an ophathalmic tonometer (Model 30 Classic, Solan). A test solution was applied to the left eye, and the right eye was not treated. After 2, 4, and 6 hours from the instillation, each rabbit was topically anesthetized with the an ophthalmologic local anesthesant, and intraocular pressure values of the both eyes were measured. Difference of the measured values of intraocular pressure of the right and left eyes at each time was regarded as intraocular pressure reduction value (overage ± standard error).

### 3. Results

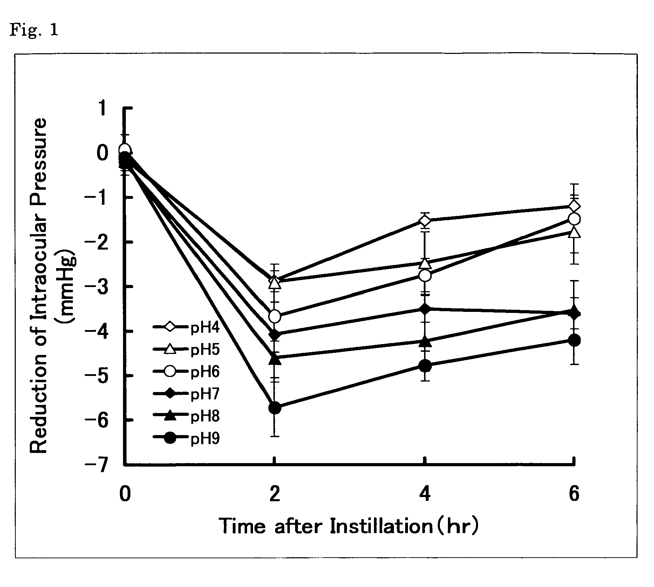

The results are shown in Fig. 1. As clearly understood from the results shown in Fig. 1, it was revealed that the intraocular pressure reducing action of the (S)-1-(4-chloro-5-isoquinolinesulfonyl)-3-(methylamino)pyrrolidine solution was more strongly exhibited when a pH became higher in the range of pH 4 to 9, and in particular, it was found that the effect was strongly exhibited at pH 7 to 9.

### [Example 5] Preparation and packaging of (S)-1-(4-chloro-5-isoquinolinesulfonyl)-3-(methylamino)pyrrolidine monohydrochloride aqueous solution

In the following examples, there were used Bankoku No. 0 Genshoku (elementary color) (Umano Kagaku Yoki K.K., Yamayu-Umano) as a white light-shielding container, Bankoku No. 1 Otaishoku (Yellow body color) (Umano Kagaku Yoki K.K., Yamayu-Umano) as a yellow light-shielding container, Bankoku No. 1 Ruri (lapis lazuli) (Umano Kagaku Yoki K.K., Yamayu-Umano) as a blue light-shielding container, Transparent UV resistant pillow bag (Okada Shigyo Co., Ltd.) as a transparent ultraviolet-shielding bag, Transparent UV resistant pillow bag (Okada Shigyo Co., Ltd.) as an orange ultraviolet-shielding bag, a bag made of Smoke SF-003 (Toyo Mark Manufacturing Co., Ltd.) as a gray ultraviolet-shielding bag, and a bag made of Dark Smoke SF-002 (Toyo Mark Manufacturing Co., Ltd.) as a dark gray ultraviolet-shielding bag.

### (1) White light-shielding container

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (50 mg) was added with the solvent (100 mL) and dissolved. This solution (2 mL) was filled in the white light-shielding container.

### (2) Yellow light-shielding container

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (20 mg) was added with the solvent (100 mL) and dissolved. This solution (4 mL) was put into the yellow light-shielding container, and the container was covered with a cap.

### (3) Blue light-shielding container

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (20 mg) was added with the solvent (100 mL) and dissolved. This solution (4 mL) was put into the blue light-shielding container, and the container was covered with a cap.

### (4) Transparent glass bottle and transparent ultraviolet-shielding bag

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (50 mg) was added with the solvent (100 mL) and dissolved. This solution (5 mL) was put into a transparent glass bottle, the bottle was covered with a cap, and put into the transparent ultraviolet-shielding bag, and the bag was sealed.

### (5) White light-shielding container and transparent ultraviolet-shielding bag

The white light-shielding container in which the (S)-1-(4-chloro-5-isoquinolinesulfonyl)-3-(methylamino)pyrrolidine monohydrochloride solution was filled in Example 5, (1) was put into the transparent ultraviolet-shielding bag, and the bag was sealed.

### (6) Transparent glass bottle and orange ultraviolet-shielding bag

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (50 mg) was added with the solvent (100 mL) and dissolved. This solution (5 mL) was put into a transparent glass bottle, the bottle was covered with a cap, and put into the orange ultraviolet-shielding bag, and the bag was sealed.

### (7) White light-shielding container and orange ultraviolet-shielding bag

The white light-shielding container in which the (S)-1-(4-chloro-5-isoquinolinesulfonyl)-3-(methylamino)pyrrolidine monohydrochloride solution was filled in Example 5, (1) was put into the orange ultraviolet-shielding bag, and the bag was sealed.

### (8) Transparent glass bottle and gray ultraviolet-shielding bag

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (50 mg) was added with the solvent (100 mL) and dissolved. This solution (5 mL) was put into a transparent glass bottle, the bottle was covered with a cap, and put into a gray ultraviolet-shielding bag, and the bag was sealed.

### (9) White light-shielding container and gray ultraviolet-shielding bag

The white light-shielding container in which the (S)-1-(4-chloro-5-isoquinolinesulfonyl)-3-(methylamino)pyrrolidine monohydrochloride solution was filled in Example 5, (1) was put into the gray ultraviolet-shielding bag, and the bag was sealed.

### (10) Transparent glass bottle and dark gray ultraviolet-shielding bag

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (50 mg) was added with the solvent (100 mL) and dissolved. This solution (5 mL) was put into a transparent glass bottle, the bottle was covered with a cap, and put into the dark gray ultraviolet-shielding bag, and the bag was sealed.

### (11) White light-shielding container and dark gray ultraviolet-shielding bag

The white light-shielding container in which the (S)-1-(4-chloro-5-isoquinolinesulfonyl)-3-(methylamino)pyrrolidine monohydrochloride solution was filled in Example 5, (1) was put into the dark gray ultraviolet-shielding bag, and the bag was sealed.

### [Comparative Example 3] Preparation and packaging of (S)-1-(4-chloro-5-isoquinolinesulfonyl)-3-(methylamino)pyrrolidine monohydrochloride aqueous solution (2)

Sodium chloride (1.8 g) was dissolved in distilled water (200 mL) to afford a solvent. (S)-1-(4-chloro-5-isoquinolinesulfonyl)-3-(methylamino)pyrrolidine monohydrochloride (20 mg) was added with the solvent (100 mL) and dissolved. This solution (5 mL) was put into a transparent glass bottle, and the bottle was covered with a cap.

### [Test Example 5] Photostability test

In the following test, LT-120D3CJ (Nagano Science Co., Ltd.) was used as a photostability tester. Purity of (S)-1-(4-chloro-5-isoquinolinesulfonyl)-3-(methylamino)pyrrolidine in the samples of the examples and the comparative example was measured by the liquid chromatography method described in Test Example 2.
The results are shown in Table 6.
The samples of Example 5 and Comparative Example 3 were subjected to light irradiation at 5,000 lux for ten days (1,200,000 lux) at 25°C by using a D65 daylight fluorescent lamp installed in the photostability tester. As the photostability tester, an apparatus was used that was capable of uniformly irradiating the samples with light by turning a table. The illumination was 5,000 lux, and 1,200,000 lux ·hr in total was irradiated over ten days. The accumulated near-ultraviolet energy during the photostability test was 474 to 488 W ·h/m². 2) Purities of (S)-1-(4-chloro-5-isoquinolinesulfonyl)-3-(methylamino)pyrrolidine in the solutions of the light-irradiated samples were compared with that of a sample stored at 5°C in a dark place. The results are shown in Table 6.

**[Table 6]**

| Package | Storage at 5°C in dark place | Light irradiation |
|---|---|---|
| Example 5, (1): White light-shielding container | 99.86% | 81.55% |
| Example 5, (2): Yellow light-shielding container | 99.85% | 69.40% |
| Example 5, (3): Blue light-shielding container | 99.84% | 71.01% |
| Example 5, (4): Transparent glass bottle and transparent ultraviolet-shielding bag | 99.74% | 97.94% |
| Example 5, (5): White light-shielding container and transparent ultraviolet-shielding bag | 99.80% | 98.78% |
| Example 5, (6): Transparent glass bottle and orange ultraviolet-shielding bag | 99.74% | 98.94% |
| Example 5, (7): White light-shielding container and orange ultraviolet-shielding bag | 99.80% | 99.18% |
| Example 5, (8): Transparent glass bottle and gray ultraviolet-shielding bag | 99.74% | 99.37% |
| Example 5, (9): White light-shielding container and gray ultraviolet-shielding bag | 99.80% | 99.61% |
| Example 5, (10): Transparent glass bottle and gray ultraviolet-shielding bag | 99.74% | 99.57% |
| Example 5, (11): White light-shielding container and dark gray ultraviolet-shielding bag | 99.80% | 99.64% |
| Comparative Example 3: Transparent glass bottle | 99.80% | 63.36% |

In all of the examples, the purity of (S)-1-(4-chloro-5-isoquinolinesulfonyl)-3-(methylamino)pyrrolidine after the light irradiation was improved as compared with that of the comparative example. It was also found that, by using a light-shielding container or ultraviolet-shielding bag having relatively low permeability for ultraviolet lights of 350 nm or shorter, orby using the a light-shielding container in combination with the ultraviolet-shielding bag, purity of (S)-1-(4-chloro-5-isoquinolinesulfonyl)-3-(methylamino)pyrrolidine after light irradiation was improved.

From the above results, it was found that where an aqueous solution containing (S)-1-(4-chloro-5-isoquinohnesulfonyl)-3-(methylamino)pyrrolidine or a pharmacologically acceptable salt thereof is filled in a container having a permeability of 40% or lower for ultraviolet lights of 350 nm or shorter, or by putting a container filled with an aqueous solution containing (S)-1-(4-chloro-5-isoquinolinesulfonyl)-3-(methylamino)pyrrolidine or a pharmacologically acceptable salt thereof into a bag having a permeability of 10% or lower for ultraviolet lights of 350 nm or shorter, the aqueous solution can be stabilized. It was also found that where a container or bag which more highly shields ultraviolet lights of 350 nm or shorter is used, or such a light-shielding container and ultraviolet-shielding bag are used in combination, an aqueous solution containing (S)-1-(4-chloro-5-isoquinolinesulfonyl)-3-(methylamino)pyrrolidine or a pharmacologically acceptable salt thereof can be further stabilized.

By using hydrochloride of (S)-1-(4-chloro-5-isoquinolinesulfonyl)-3-aminopyrrolidine obtained according to the method described in International Patent Publication W02007/026664 in Test Example 5, instead of (S)-1-(4-chloro-5-isoquinolinesulfonyl)-3-(methylamino)pyrrolidine monohydrochloride, a photostability test of an aqueous solution containing (S)-1-(4-chloro-5-isoquinolinesulfonyl)-3-aminopyrrolidine or a pharmacologically acceptable salt thereof can be performed.

In addition, as for an aqueous solution containing (S)-1-(4-chloro-5-isoquinolinesulfonyl)-3-aminopyrrolidine or a pharmacologically acceptable salt thereof, since absorption spectrum of said substance is similar to that of (S)-1-(4-chloro-5-isoquinolinesulfonyl)-3-(methylamino)pyrrolidine or a pharmacologically acceptable salt thereof, it is estimated that photostability properties of said substance are similar to those of (S)-1-(4-chloro-5-isoquinolinesulfonyl)-3-(methylamino)pyrrolidine or a pharmacologically acceptable salt thereof.

### Industrial Applicability

The aqueous solution composition of the present invention has superior stability for long-term storage, and has an enhanced intraocular pressure reducing action. Accordingly, the composition is preferred as an intraocular pressure reducing agent, especially as a medicament for instillation.

## Claims

1. An aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, wherein citric acid or a salt thereof is not contained.

2. The aqueous solution composition according to claim 1, which contains (S)-1-(4-chloro-5-isoquinolinesulfonyl)-3-aminopyrrolidine or a pharmacologically acceptable salt thereof as an active ingredient.

3. The aqueous solution composition according to claim 1, which contains (S)-1-(4-chloro-5-isoquinolinesulfonyl)-3-(methylamino)pyrrolidine or a pharmacologically acceptable salt thereof as an active ingredient.

4. The aqueous solution composition according to any one of claims 1 to 3, wherein the pharmacologically acceptable salt is a hydrogen halide acid salt.

5. The aqueous solution composition according to claim 4, wherein the pharmacologically acceptable salt is monohydrochloride.

6. The aqueous solution composition according to any one of claims 1 to 5, wherein the aqueous solution has a pH of 5 to 9.

7. The aqueous solution composition according to claim 6, which further contains a pH adjustor other than citric acid or a salt thereof.

8. The aqueous solution composition according to claim 7, wherein the pH adjustor other than citric acid or a salt thereof consists of one kind or two or more kinds of pH adjustors selected from the group consisting of phosphoric acid and a salt thereof, acetic acid and a salt thereof, tris(hydroxymethyl)aminomethane and a salt thereof, as well as boric acid and a salt thereof.

9. The aqueous solution composition according to claim 7 or 8, wherein a ratio of molar concentration of the pH adjustor and a total of molar concentrations of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof is 1:10 to 200:1.

10. An aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, wherein the aqueous solution has a pH of 7 to 9.

11. The aqueous solution composition according to claim 10, wherein the aqueous solution has a pH of 7 to 9.

12. A method for stabilizing an aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, which comprises the step of adjusting a pH of the aqueous solution composition to 5 to 9 by using a pH adjustor other than citric acid or a salt thereof in the absence of citric acid or a salt thereof.

13. A method for preparing a long-term storable aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, which comprises the step of adjusting a pH of the aqueous solution composition to 5 to 9 by using a pH adjustor in the absence of citric acid or a salt thereof.

14. A method for preparing a pharmaceutical composition wherein intraocular pressure reducing action of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof is enhanced, which comprises preparing the substance as an aqueous solution composition having a pH of 7 to 9.

15. A method for enhancing intraocular pressure reducing action of one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof, which comprises using the substance as an aqueous solution having a pH of 7 to 9.

16. A method for preparing a long-term storable aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof having enhanced intraocular pressure reducing action, which comprises preparing the substance as an aqueous solution composition having a pH of 7 to 9 by using a pH adjustor in the absence of citric acid or a salt thereof.

17. An aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient, which is filled in a container having a permeability of 40% or lower for ultraviolet lights having a wavelength of 350 nm or shorter, and/or filled in a container stored in a packaging container having a permeability of 20% or lower for ultraviolet lights having a wavelength of 350 nm or shorter.

18. The aqueous solution composition according to any one of claims 1 to 17, which is filled in a container having a permeability of 40% or lower for ultraviolet lights having a wavelength of 350 nm or shorter, and/or filled in a container stored in a packaging container having a permeability of 20% or lower for ultraviolet lights having a wavelength of 350 nm or shorter.

19. The aqueous solution composition according to claim 18, wherein the container is a container for instillation.

20. The aqueous solution composition according to claim 18 or 19, wherein the container in which the aqueous solution composition is filled is made of plastics.

21. A method for stabilizing an aqueous solution composition containing one kind or two or more kinds of substances selected from the group consisting of (S)-1-(4-chloro-5-isoquinolinesulfonyl)-3-aminopyrrolidine and a pharmacologically acceptable salt thereof, and (S)-1-(4-chloro-5-isoquinolinesulfonyl)-3-(methylamino)pyrrolidine and a pharmacologically acceptable salt thereof as an active ingredient against light, which comprises the step of filling the aqueous solution composition in a container having a permeability of 40% or lower for ultraviolet lights having a wavelength of 350 nm or shorter, and/or the step of filling the aqueous solution composition in a container stored in a packaging container having a permeability of 20% or lower for ultraviolet lights having a wavelength of 350 nm or shorter.

22. A method for stabilizing the aqueous solution composition according to any one of claims 1 to 11 against light, which comprises the step of filling the aqueous solution composition in a container having a permeability of 40% or lower for ultraviolet lights having a wavelength of 350 nm or shorter, and/or the step of filling the aqueous solution composition in a container stored in a packaging container having a permeability of 20% or lower for ultraviolet lights having a wavelength of 350 nm or shorter.
